(19) Europäisches Patentamt
European Patent Office
Office européen des brevets

(11) EP 2 596 788 A1

(12) EUROPEAN PATENT APPLICATION
published in accordance with Art. 153(4) EPC

(43) Date of publication:
29.05.2013 Bulletin 2013/22

(51) Int Cl.:
A61K 31/436 (2006.01)    A61K 9/107 (2006.01)
A61K 47/14 (2006.01)

(21) Application number: 11809736.9

(22) Date of filing: 22.07.2011

(86) International application number:
PCT/JP2011/066698

(87) International publication number:
WO 2012/011566 (26.01.2012 Gazette 2012/04)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 23.07.2010 PCT/JP2010/062467

(71) Applicant: Maruho Co., Ltd.
Osaka 531-0071 (JP)

(72) Inventors:
• IHARA, Mikito
Kyoto-shi
Kyoto 600-8815 (JP)
• UEDA, Yoshinori
Kyoto-shi
Kyoto 600-8815 (JP)

(74) Representative: Pfenning, Meinig & Partner GbR
Theresienhöhe 13
80339 München (DE)

(54) TACROLIMUS-CONTAINING OIL-IN-WATER TYPE CREAMY COMPOSITION

(57) The purpose is to provide a tacrolimus-containing pharmaceutical composition which is a creamy preparation for external application having good feeling upon use and has high stability of a main ingredient contained therein (high main ingredient residual ratio), and allows easy control of skin concentration of the main ingredient.

The present invention relates to an oil-in-water type creamy composition comprising (A) tacrolimus, a pharmaceutically acceptable salt thereof, or a pharmaceutically acceptable solvate thereof, (B) an oil prepared by mixing (a) a medium-chain fatty acid triglyceride with (b) ethylene glycolsalicylate and/or diisopropylsebacate, (C) an emulsifying agent having a HLB value of 12 or more, and (D) a hydrophilic polymer, and having a pH value of 4 to 7.

EP 2 596 788 A1

**Description**

TECHNICAL FIELD

The present invention relates to an oil-in-water type (O/W) creamy composition containing tacrolimus.

BACKGROUND ART

Tacrolimus preparations for external application are known to have excellent therapeutic effect on atopic dermatitis, and currently, Protopic (registered trademark) ointment 0.1% and Protopic (registered trademark) ointment 0.03% for pediatric use are commercially available in the form of oily ointment containing an oily base. However, oily ointment is poor feeling upon use at the time of application on the skin due to their stickiness despite its merit such as excellent skin protecting action, and hence creamy preparations for external application having better feeling upon use are demanded by patients and health care workers.

A creamy preparation for external application is produced by using an emulsion base that is obtained by emulsifying oil and water with an emulsifying agent. Since tacrolimus is unstable to water, when an emulsion base is used, the residual ratio of tacrolimus is lower than that in the case of using an oily base, leading the problem that it is technically difficult to maintain the efficacy (concentration in skin).

For this, preparation of a creamy pharmaceutical composition has been attempted (see Patent Document 1), however, a creamy pharmaceutical composition that is excellent in every points including feeling upon use, the stability of tacrolimus in the preparation, and transdermal absorption has not been marketed up to today.

Accordingly, there is still a demand for a composition, in a preparation for external application containing tacrolimus as a main ingredient, which is in a creamy state realizing good feeling upon use, provides good stability of the main ingredient in the preparation, and is able to make concentration in the skin of tacrolimus within an optimum range.

PRIOR ART DOCUMENTS

PATENT DOCUMENTS

Patent Document 1: JP 2000-513739 W

SUMMARY OF THE INVENTION

PROBLEMS TO BE SOLVED BY THE INVENTION

Therefore, it is an object of the present invention to provide a tacrolimus-containing pharmaceutical composition which is a creamy preparation for external application having good feeling upon use and realizes high stability of tacrolimus (high main ingredient residual ratio) in the preparation, and is able to achieve desired transdermal absorption in the site where it is applied.

MEANS FOR SOLVING THE PROBLEMS

To solve the above problem, inventors of the present invention made repeated studies for an oil having excellent dissolubility of tacrolimus and capable of keeping tacrolimus stable (hereinafter, also referred to as a dissolving agent), and found that the aforementioned problem can be effectively solved by dissolving tacrolimus in an oil obtainable by mixing specific dissolving agents, and preparing an oil-in-water type composition having the oil as an inner phase (oil phase), and accomplished the present invention.

That is, the present invention is an oil-in-water type creamy composition containing:

(A) tacrolimus, a pharmaceutically acceptable salt thereof, or a pharmaceutically acceptable solvate thereof;
(B) an oil prepared by mixing (a) a medium-chain fatty acid triglyceride with (b) ethylene glycol salicylate and/or diisopropyl sebacate;
(C) an emulsifying agent having an HLB value of 12 or more; and
(D) a hydrophilic polymer, and
having a pH value of 4 to 7.

Since the creamy composition according to the present invention is of an oil-in-water type having an oil phase as an inner phase, and contains tacrolimus, a salt thereof, or a solvate thereof (hereinafter, these are called tacrolimus as a representative), which is a main ingredient in the oil phase, it is possible to reduce contact between the main ingredient and the water phase (outer phase), and to keep tacrolimus that is unstable to water stable. Also, by using a combination of (a) a medium-chain fatty acid triglyceride and (b) ethylene glycol salicylate and/or diisopropyl sebacate as the oil phase, it is possible to obtain an oil-in-water type (O/W type) creamy composition that is excellent in any of the dissolubility of tacrolimus, the stability of tacrolimus in the preparation, and transdermal absorption of tacrolimus. Also, by using an emulsifying agent having a HLB value of 12 or more, and a hydrophilic polymer, it is possible to achieve excellent dispersibility, and by realizing a pH value of 4 to 7, it is possible to keep tacrolimus stable.

EFFECT OF THE INVENTION

According to the present invention, it is possible to provide a creamy composition capable of keeping tacrolimus in a preparation stable, and exerting excellent drug efficacy, and having good feeling upon use (little stickiness).

BRIEF DESCRIPTION OF THE DRAWINGS

Fig. 1 is a graph showing a result of in vitro skin permeability test for tacrolimus (in human skin).
Fig. 2 is a graph showing a result of in vitro skin permeability test for tacrolimus (in human corneum).
Fig. 3 is a graph showing a result of a drug efficacy test using a mouse contact dermatitis model.
Fig. 4 shows a chart (example) of a rheometer.

MODE FOR CARRYING OUT THE INVENTION

Preferably, the creamy composition of the present application contains 0.01 to 0.3% by weight of tacrolimus. When the content of tacrolimus is less than 0.01% by weight, efficacy is poor, and when it is more than 0.3% by weight, there is fear for the safety.
As a pharmaceutically acceptable salt of tacrolimus, nontoxic, pharmaceutically acceptable commonly-used salts can be used. Examples of such salts include salts with an inorganic or an organic base such as alkaline metal salts (sodium salt, potassium salt and so on), alkaline earth metal salts (calcium salt, magnesium salt and so on), ammonium salts, and amine salts (triethylamine salt, N-benzyl-N-methylamine salt and so on).
As a pharmaceutically acceptable solvate of tacrolimus, hydrates and ethanolates are recited.
In the creamy composition of the present application, as an oil that forms an oil phase, both (a) a medium-chain fatty acid triglyceride (hereinafter, also referred to as MCT) and (b) ethylene glycol salicylate (hereinafter, also referred to as EGS) and/or diisopropyl sebacate (hereinafter, also referred to as IPSE) are used. In other words, a mixture of the (a) and (b) is used as the oil. In the present invention, the oil that forms the oil phase is substantially composed of a mixture of the (a) and (b). The term "substantially" means that a total amount of the (a) and (b) occupies 95% by weight or more (more preferably, 97% by weight or more, and particularly preferably 99% by weight or more) of the total amount of the oil.
MCT of (a) is a compound wherein three molecules of $C_6$-$C_{12}$ saturated fatty acid are ester-linked with glycerin. In the present invention, triglyceride of $C_8$-$C_{10}$ saturated fatty acid is particularly preferred.
EGS and IPSE of (b) may be used singly or in combination. From the view point of improving the transdermal absorption, it is preferred to use EGS singly or to use both EGS and IPSE while the rate of EGS is made higher.
The total amount of (a) and (b) in the total amount of the composition is preferably 1 to 50% by weight. When it is less than 1% by weight, the stability and the absorption of tacrolimus are impaired, whereas when it is more than 50% by weight, it is difficult to prepare an oil-in-water type creamy composition. A more preferred content is 8 to 45% by weight, and a particularly preferred content is 10 to 30% by weight.
The amount of (a) in the total amount of the composition is preferably 0.5 to 49.5% by weight. When it is less than 0.5% by weight, the stability of tacrolimus is impaired, whereas when it is more than 49.5% by weight, it is difficult to prepare an oil-in-water type creamy composition. A more preferred content is 5 to 30% by weight, and a particularly preferred content is 5 to 20% by weight.
The amount of (b) in the total amount of the composition is preferably 0.5 to 20% by weight. When it is less than 0.5% by weight, the stability and the absorption of tacrolimus are impaired, whereas when it is more than 20% by weight, it is difficult to prepare an oil-in-water type creamy composition. A more preferred content is 3 to 15% by weight, and a particularly preferred content is 5 to 15% by weight.
When both (a) and (b) are used, a higher rate of (a) gives merits of increasing emulsifying property and increasing the range of choices for the emulsifying agent, and facilitating preparation of an oil-in-water type creamy composition, however, the stability of the main ingredient in the oil-in-water type creamy composition tends to deteriorate because the dissolubility of tacrolimus is decreased.

On the other hand, a higher rate of (b) gives merits of increasing the stability of the main ingredient in the oil-in-water type creamy composition because the dissolubility of tacrolimus increases, however, preparation of an oil-in-water type creamy composition tends to be difficult because emulsifying property is decreased (in particular when EGS is used) and the range of choices for the surfactant is decreased.

Also, according to the present invention, by varying the ratio between (a) and (b), the transdermal absorption of tacrolimus can be controlled, and by increasing the rate of (a), the transdermal absorption can be controlled to a lower level, and by increasing the rate of (b), the transdermal absorption can be controlled to a higher level.

To be more specific about control of transdermal absorption, a composition having higher transdermal absorption of tacrolimus is naturally desired when high drug efficacy is expected. On the other hand, since tacrolimus has strong immunosuppressive action, incidence of side effect can be increased due to increase in blood concentration. For example, when it is applied on the skin whose transdermal absorption is increased due to decrease in barrier function of the corneum, or when it is applied on a specific patient (for example, child, pregnant woman, nursing women, elderly, patient suffering from hepatic disorder and so on), a preparation with reduced absorption can be rather requested.

In the present invention, by adjusting the ratio between (a) and (b) that form the oil phase, or the kind or the rate of (b) (EGS and/or IPSE), it is possible to control the absorption to the skin while keeping the high preparation stability, so that it is possible to respond to these requests.

While the use ratio between (a) and (b) may be appropriately determined in accordance with desired preparation characteristics in consideration of the aforementioned points, generally, the ratio between (a) and (b) (weight ratio) a : b is adjusted preferably within the range of 20 : 1 to 1 : 20, and more preferably within the range of 5 : 1 to 1 : 5. Particularly, by setting the ratio within the range of 2 : 1 to 1 : 2, it is possible to obtain a composition having a good balance among the emulsifying property, the main ingredient stability, and the transdermal absorption.

The creamy composition according to the present invention contains, as an emulsifying agent (surfactant) for emulsifying a water phase and an oil phase and stabilizing the same, an emulsifying agent (C) having a HLB value of 12 or more. With an emulsifying agent having a HLB value of less than 12, it is difficult to prepare an oil-in-water type creamy composition having excellent emulsion stability. A more preferred emulsifying agent is an emulsifying agent having a HLB value of 12 to 18.

The content of the emulsifying agent is preferably 0.1 to 10% by weight. When it is more than 10% by weight, there would be fear for the stability of the preparation, whereas when it is less than 0.1% by weight, it is difficult to produce a stable oil-in-water type creamy composition. A more preferred content of the emulsifying agent is 0.5 to 8% by weight, and 3 to 7% by weight is particularly preferred.

The emulsifying agent may be used singly or in combination of plural kinds. Concrete examples of a preferred emulsifying agent include polyoxyethylene hydrogenated castor oil, polyoxyethylene polyoxypropylene alkyl ether and polyglycerin fatty acid ester, and as a particularly preferred emulsifying agent, polyoxyethylene hydrogenated castor oil (for example, polyoxyethylene hydrogenated castor oil (60)) is recited.

The creamy composition according to the present invention contains a hydrophilic polymer (D) for thickening the composition and ensuring the stability as a preparation (preventing separation). The content of the hydrophilic polymer is preferably 0.1 to 10% by weight. When it is more than 10% by weight, the preparation is so hard that usability can be impaired, whereas when it is less than 0.1% by weight, it is difficult to produce a stable oil-in-water type creamy composition. A more preferred content of the hydrophilic polymer is 0.2 to 2% by weight.

Concrete examples of a preferred hydrophilic polymer include water-soluble cellulose derivatives such as carboxyvinyl polymer, hydroxyethyl cellulose, hydroxypropyl cellulose, hydroxypropyl methyl cellulose, hydrophobized hydroxypropyl methyl cellulose and the like. These may be used singly or in combination. As a particularly preferred hydrophilic polymer, carboxyvinyl polymer is recited.

The creamy composition according to the present invention has a pH value ranging from 4 to 7. When the pH value is less than 4, there can be fear for the safety as a preparation, whereas when the pH value is more than 7, there is fear for hydrolysis of the oil (a) and (b). The aforementioned pH value is also optimum pH in the aspect of the stability of tacrolimus. A more preferred pH range is 5 to 6.

As a pH modifier for adjusting the pH value of the composition within the aforementioned range, lactic acid, citric acid, phosphoric acid and so on are recited as those used for adjusting to a low pH region, and sodium hydroxide, potassium hydroxide, sodium lactate, sodium citrate, L-arginine, diisopropanolamine and so on are recited as those used for adjusting to a high pH region.

The creamy composition according to the present invention may contain a wetting agent, a preservative, a stabilizing agent, an emulsification auxiliary agent and so on, besides the aforementioned ingredients.

Examples of the wetting agent include 1, 3-butylene glycol, glycerin, propylene glycol and dipropylene glycol. Particularly preferred wetting agents include 1,3-butylene glycol. The content of the wetting agent in the composition is preferably within the range of 0.01 to 30% by weight, and more preferably within the range of 1 to 20% by weight.

Examples of the preservative include methyl paraoxybenzoate, ethyl paraoxybenzoate, propyl paraoxybenzoate, sodium benzoate and phenoxyethanol. The content of the preservative in the composition is preferably within the range of 0.01 to 2% by weight, and more preferably within the range of 0.1 to 1.0% by weight. When it is more than 2% by weight, there can be fear for the safety of a preparation, and when it is less than 0.01% by weight, it is difficult to exert sufficient preservation effect.

Examples of the stabilizing agent include dibutyl hydroxy toluene, pentaerythrityl-tetrakis

[3-(3,5-di-t-butyl-4-hydroxyphenyl)propionate], sodium thiosulfate hydrate, propyl gallate, ascorbic acid, tocopherol, sodium edetate hydrate, sodium metaphosphate, benzotriazole and 2-mercaptobenzimidazole. The content of the stabilizing agent in the composition is preferably within the range of 0. 005 to 2% by weight, and more preferably within the range of 0.01 to 0.2% by weight. When it is more than 2% by weight, there can be fear for the safety of a preparation, and when it is less than 0.005% by weight, it is difficult to exert sufficient stabilizing effect.

Examples of the emulsification auxiliary agent include cetanol, stearyl alcohol, cetostearyl alcohol, and behenyl alcohol. As a particularly preferred emulsification auxiliary agent, cetanol is recited. The content of the emulsification auxiliary agent in the composition is preferably within the range of 0.1 to 5% by weight as is necessary, and more preferably within the range of 0.5 to 3% by weight.

The creamy composition of the present invention contains water (purified water) that forms the water phase. The content of the water in the composition varies depending on the contents of other ingredients, however, it is preferably about 50 to 75% by weight, and more preferably about 55 to 70% by weight.

In the following, the present invention will be described more specifically by Examples, however, the present invention is not limited by these examples.

EXAMPLES

(1) Examination of dissolving agent for tacrolimus hydrate

For the purpose of finding a dissolving agent that is excellent in the dissolubility and the stability of tacrolimus hydrate, various oils were tested.
First, solubility of tacrolimus hydrate in low polar to high polar oil that has been previously used as a general preparation for external application was evaluated. As a result of tests for a large number of oils, eight oils exhibited the dissolubility of 0.5% or more with respect to tacrolimus hydrate.

Next, the eight oils were evaluated for the stability of tacrolimus hydrate by the method as shown below.

[Stability test]

A maximum amount of tacrolimus hydrate was dissolved in each of various oils (ethylene glycol salicylate, propylene carbonate: 50 mg/g, crotamiton, triacetin, macrogol 400, 1,3-butyleneglycol: 20 mg/g, diisopropyl sebacate: 10 mg/g, medium-chain fatty acid triglyceride: 5 mg/g) to give an oil solution. This was stored under the condition of 60°C/ambient humidity, and the content of tacrolimus hydrate was time-dependently measured according to "The Japanese Pharmacopoeia Fifteenth Edition, General Tests, Liquid Chromatography <2.01>", and a residual ratio was calculated according to the following Formula 1.

[Formula 1]

$$\text{Residual ratio of tacrolimus hydrate (\%)} = \text{content of tacrolimus hydrate in stored article (\%)/content of tacrolimus hydrate in initial article (\%)} \times 100$$

The result of the stability test is shown in Table 1. The stability was highest in a medium-chain fatty acid triglyceride, and second highest in diisopropyl sebacate, crotamiton and ethylene glycol salicylate. In the present example, a medium-chain fatty acid triglyceride wherein medium-chain fatty acid is caprylic acid and capric acid was used.

[Table 1]

Table 1. Result of stability of main ingredient in oil (60°C/ambient humidity, n=1*1 or 2*2)

| Oil (tacrolimus hydrate solubility) | Residual ratio of tacrolimus hydrate, unit: % | | | |
|---|---|---|---|---|
| | Initial | 1 week | 2 weeks | 4 weeks |
| Ethylene glycol salicylate(5%)*1 | 100 | 97.5 | 93.7 | 88.3 |
| Crotamiton(2%)*1 | 100 | 98.0 | 96.0 | 92.0 |
| Triacetin(2%)*1 | 100 | 93.3 | 77.1 | 47.5 |
| Diisopropyl sebacate(1%)*1 | 100 | 99.1 | 96.3 | 92.2 |
| Propylene carbonate(5%)*2 | 100 | 100.5 | 94.0 | 56.2 |
| Macrogol 400(2%)*2 | 100 | 95.5 | 92.4 | 71.9 |
| 1,3-butyleneglycol(2%)*2 | 100 | 91.6 | 86.6 | 63.1 |
| Middle-chain fatty acid triglyceride (0.5%)*2 | 100 | 98.6 | 98.8 | 102.2 |

Considering the dissolubility and the stability, MCT was selected as a candidate dissolving agent. Also the oil that exhibited a main ingredient residual ratio after 4 weeks of 85% or more and having higher dissolubility of tacrolimus hydrate than MCT was selected as a candidate dissolving agent.

(2) Examination of emulsifying agent

Using the candidate dissolving agents selected in the above (1), an appropriate emulsifying agent for preparing an oil-in-water type (O/W type) creamy composition was examined.
Using various emulsifying agents, an O/W type creamy composition containing 20% by weight of MCT, and an O/W type creamy composition containing each of 10% by weight of MCT and EGS was prepared. The creamy compositions were prepared according to a later-described method (3-1).
The results are shown in Tables 2-1 and 2-2. As to whether or not formulation of a creamy composition is possible, "×" is given to the emulsifying agent in which separation or the like was observed in appearance of the creamy composition, and "O" is given to the emulsifying agent in which separation or the like was not observed.
[Table 2]

Table 2-1. Emulsifying agent used in examination for formulation of 20% MCT-containing O/W type creamy composition

| Ingredient name | HLB | Possibility of formulation |
|---|---|---|
| Self-emulsifying type glycerin monostearate | 8.0 | × |
| Polyoxyethylene stearyl ether | 9.0 | O |
| Polyoxyethylene glycerin monostearate | 9.5 | O |
| Polyoxyethylene cetyl ether | 10.5 | O |
| Polyoxyethylene sorbitan tristearate | 10.5 | O |
| Polyethylene glycol monostearate | 11.0 | O |
| Lauromacrogol | 11.5 | O |
| Decaglyceryl monostearate | 12.0 | O |
| Polyoxyethylene(20)polyoxypropylene(8) cetyl ether | 12.5 | O |
| Polyoxyethylene hydrogenated castor oil 40 | 12.5 | O |

Table 2-2. Emulsifying agent used in examination for formulation of 10% MCT+10% EGS-containing O/W type creamy composition

| Ingredient name | HLB | Possibility of formulation |
|---|---|---|
| Polyoxyethylene glycerin monostearate | 9.5 | × |
| Decaglyceryl monostearate | 12.0 | ○ |
| Polyoxyethylene(20) polyoxypropylene(8)cetyl ether | 12.5 | ○ |
| Polyoxyethylene hydrogenated castor oil 40 | 12.5 | ○ |
| Polyoxyethylene hydrogenated castor oil 60 | 14.0 | ○ |

As a result of the above test, as shown in Table 2-1, in the O/W type creamy composition containing 20% MCT, a good composition could be prepared when an emulsifying agent having a HLB value of 9 or more was used. On the other hand, as shown in Table 2-2, in the O/W type creamy composition containing 10% MCT + 10% EGS, a good composition could be prepared when an emulsifying agent having a HLB value of 12 or more was used.

<u>(3) Preparation and stability evaluation of O/W type creamy composition containing tacrolimus hydrate</u>

Using the candidate dissolving agents selected based on the examination of the above (1), and the emulsifying agent selected based on the examination of the above (2), O/W type creamy compositions containing tacrolimus hydrate were prepared and the stability thereof were evaluated. In the following, a preparation method of an O/W type creamy composition, and a stability evaluation method will be described.

(3-1) Preparation method of O/W type creamy composition (one example)

Carboxyvinyl polymer was preliminarily swelled with purified water, to give a carboxyvinyl polymer aqueous solution. Also, diisopropanolamine was dissolved in purified water, to give a diisopropanolamine aqueous solution. In accordance with a prescription, tacrolimus hydrate, dibutyl hydroxy toluene, propyl paraoxybenzoate and cetanol were dissolved in oil, and added with an emulsifying agent to give an oil phase. On the other hand, a carboxyvinyl polymer aqueous solution, 1,3-butylene glycol, methyl paraoxybenzoate, sodium edetate hydrate and purified water were combined to give a water phase.
After dissolving the oil phase and the water phase by heating to 70 to 90°C, respectively, the water phase was added to the oil phase, and emulsified by using a homomixer. Then, after adding a diisopropanolamine aqueous solution, the product was stirred with a stirrer bar until the product temperature reaches room temperature, to produce a homogeneous cream agent.
It is to be noted that the preparation method of an O/W type creamy composition is not limited to this.

(3-2) Evaluation of stability of tacrolimus hydrate

A prepared creamy composition was stored in a predetermined condition, and a tacrolimus hydrate content was time-dependently measured according to "The Japanese Pharmacopoeia Fifteenth Edition, General Tests, Liquid Chromatography <2.01>", and a residual ratio of tacrolimus hydrate in the creamy composition was calculated according to the foregoing Formula 1 and the stability of the main ingredient in the composition was evaluated.

The stability test of creamy composition revealed that higher stability (residual ratio) of tacrolimus hydrate was realized by the creamy composition prepared by using a mixture of MCT and a specific oil (concretely, IPSE or EGS), rather than that prepared by using only MCT that exhibited the highest main ingredient stability in the aforementioned (1) "Examination of dissolving agent".

The results are collectively shown in Table 3 and Table 4. Table 3 shows composition of each creamy composition and information of used reagents and so on, and Table 4 shows a measurement result of main ingredient stability in each creamy composition. The composition in which only MCT is used as an oil is called cream A, the composition in which a mixture of MCT and IPSE is used is called cream B, and the composition in which a mixture of MCT and EGS is used is called cream C. Also prescriptions based on cream C with improved stability are called creams D to G. When polyoxyethylene hydrogenated castor oil 60 (see Table 2-2) that showed particularly excellent emulsifying property on MCT + EGS in the aforementioned (2) "Examination of emulsifying agent" is used as an emulsifying agent of each creamy composition, it was possible to prepare a very good O/W type creamy composition even when only MCT (cream A), or a mixture of MCT and IPSE (cream B) is used as an oil.

[Table 3]

Table 3 Prescription of O/W type creamy composition (% by weight)

| Ingredient | | creamA | creamB | creamC | cream D | creamE | creamF | creamG |
|---|---|---|---|---|---|---|---|---|
| Tacrolimus hydrate | Main ingredient | 0.102 | 0.102 | 0.102 | 0.102 | 0.102 | 0.102 | 0.102 |
| Middle-chain fatty acid triglyceride(MCT) | Oil | 20 | 10 | 10 | 10 | 10 | 10 | 10 |
| Diisopropyl sebacate(IPSE) | Oil | - | 10 | - | - | - | - | - |
| Ethyleneglycol salicylate (EGS) | Oil | - | - | 10 | 10 | 10 | 10 | 10 |
| Polyoxyethylene hydrogenated castor oil 60 | Emulsifying agent | 5 | 5 | 5 | 5 | 5 | 5 | 5 |
| Carboxyvinyl polymer | Hydrophilic polymer | 1 | 1 | 1 | 1 | 1 | 1 | 1 |
| 1,3-butylene glycol | Wetting agent | 10 | 10 | 10 | 10 | 10 | 5 | 5 |
| Dibutylhydroxytoluene | Stabilizing agent | - | - | - | 0.1 | - | - | 0.1 |
| Propyl paraoxybenzoate | Preservative | - | - | - | - | 0.05 | 0.05 | 0.05 |
| Cetanol | Emulsification auxiliary agent | - | - | - | - | 1 | - | 1 |
| Methyl paraoxybenzoate | Preservative | 0.2 | 0.2 | 0.2 | 0.2 | 0.2 | 0.2 | 0.2 |
| Diisopropanol amine | pH modifier | 0.5 | 0.5 | 0.5 | 0.5 | 0.5 | 0.5 | 0.5 |
| Sodium edetate hydrate | Stabilizing agent | 0.02 | 0.02 | 0.02 | 0.02 | 0.02 | 0.02 | 0.02 |
| Purified water | Base | balance | balance | balance | balance | balance | balance | balance |
| Total amount | | 100 | 100 | 100 | 100 | 100 | 100 | 100 |
| pH | | 5.2 | 5.3 | 5.2 | 5.3 | 5.3 | 5.1 | 5.2 |

| Information about reagents | | |
|---|---|---|
| Name | Trade name | Manufacturer |
| Middle-chain fatty acid triglyceride | Migliol 810 | Mitsuba Trading Co., Ltd. |
| Diisopropyl sebacate | IPSE | Nippon Fine Chemical Co., Ltd. |
| Ethyleneglycol salicylate | Saliment | API Corporation |
| Polyoxyethylene hydrogenated castor oil 60 | NIKKOL HCO-60(for medical use) | Nikko Chemicals Co., Ltd. |
| Carboxyvinyl polymer | Cabopol 980 | Nikko Chemicals Co., Ltd. |
| 1,3-butylene glycol | 1,3-butyleneglycol | Daicel Corporation |
| Dibutylhydroxytoluene | BHT | Wako Pure Chemical Industries Ltd. |
| Cetanol | Cetanol (JP GRADE) | Kao Corporation |
| Methyl paraoxybenzoate | Methylparaben | Midori Kagaku Co., Ltd. |
| Propyl paraoxybenzoate | Propylparaben | Midori Kagaku Co., Ltd. |
| Diisopropanol amine | DIPA100% | Mitsui Fine Chemical Co., Ltd. |
| Sodium edetate hydrate | Clewat N | Nagase ChemteX Corporation |

[Table 4]

Table 4-1. Result of stability test of tacrolimus hydrate-containing O/W type creamy composition (n = 3)

| Storage condition | Composition | Residual ratio of tacrolimus hydrate (R.S.D.), unit: % | |
|---|---|---|---|
| | | Initial | 4 weeks |
| 40°C/75%RH | cream A | 100 (0.1) | 93.9 (1.2) |
| | cream B | 100 (0.1) | 96.4 (1.0) |
| | cream C | 100 (0.3) | 96.7 (0.5) |

Table 4-2. Result of stability test of tacrolimus hydrate-containing O/W type creamy composition (n = 3)

| Storage condition | Composition | Residual ratio of tacrolimus hydrate (R.S.D.), unit: % | |
|---|---|---|---|
| | | Initial | 6 months |
| 25°C/60%RH | cream C | 100 (0.6) | 94.5 (0.4) |
| | cream E | 100 (0.3) | 99.0 (1.4) |
| | cream F | 100 (0.5) | 98.7 (1.2) |
| | cream G | 100 (0.9) | 99.6 (0.9) |
| | P ointment | 100 (1.6) | 97.8 (0.8) |
| 30°C/65%RH | cream C | 100 (0.6) | 90.3 (0.4) |
| | cream E | 100 (0.3) | 95.8 (0.7) |
| | cream F | 100 (0.5) | 96.7 (0.9) |
| | cream G | 100 (0.9) | 95.7 (1.3) |
| | P ointment | 100 (1.6) | 96.8 (1.0) |

As shown in Table 4-1, in the composition containing only MCT as an oil (creamA), the main ingredient residual ratio after 4 weeks under the condition of 40°C/75% RH was less than 94%, whereas in the composition containing MCT and IPSE as oils (cream B) and in the composition containing MCT and EGS as oils (cream C), the main ingredient residual ratio was more than 96%.

Also, as shown in Table 4-2, creams C, E, F, G showed a comparatively high main ingredient residual ratio even when they were stored for 6 months under the conditions of 25°C/60%RH and 30°C/65%RH.

Creams C, E, F, G are compositions containing the same oils. Cream E is a composition containing cetanol, and showed a higher main ingredient residual ratio than cream C. This is attributable to the fact that tacrolimus is trapped into the oil phase due to the interface stabilizing activity of cetanol, and distribution into the water phase is suppressed. Cream F is a composition containing a smaller amount of 1,3-butyleneglycol than cream C, and showed a higher main ingredient residual ratio than cream C. This is attributable to the fact that distribution of tacrolimus into the water phase is suppressed by reduction of the amount of 1, 3-butyleneglycol. Cream G is a composition containing dibutyl hydroxy toluene and cetanol, and a smaller amount of 1, 3-butyleneglycol, and showed a higher main ingredient residual ratio than cream C. This is attributable to the fact that in addition to the effects by addition of cetanol and reduction in the amount of 1, 3-butyleneglycol as described above, dibutyl hydroxy toluene prevents oxidative degradation of tacrolimus.

As described above, even after storage for 6 months, creams E to G showed a high main ingredient residual ratio exceeding 95%, and it was demonstrated that they have main ingredient stability that is comparable to that obtainable by Protopic (registered trademark) ointment 0.1% (P ointment).

(4) Efficacy of O/W type creamy composition (Evaluation of in vitro human skin permeability)

The concentration in the skin and the amount in the corneum after 24 hours from application when the forgoing creams A to C and Protopic (registered trademark) ointment 0.1% (P ointment) were applied on a normal human skin (obtained from HAB Research Organization) were compared.

A human abdominal skin (1.77 cm$^2$) was attached to a Franz cell, and 10 mg of the foregoing cream A, B, C or P ointment was open applied. A receptor liquid (1 w/v% bovine serum albumin-containing phosphate buffer saline) was collected after a predetermined time, and a tacrolimus concentration in the skin and a tacrolimus amount in the corneum after 24 hours from application were quantified.

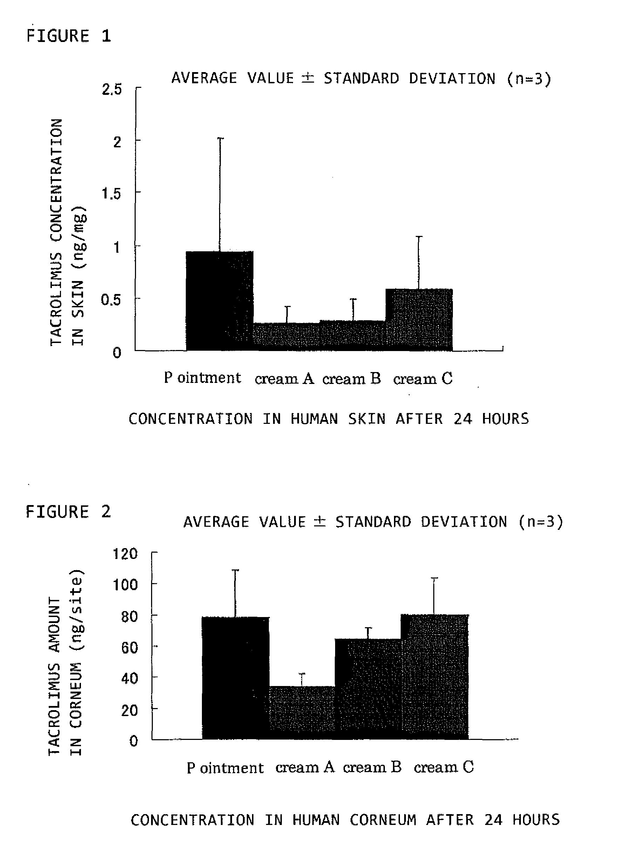

The results are shown in Fig. 1 and Fig. 2. The tacrolimus concentration in the skin and the tacrolimus amount in the corneum after 24 hours from application of cream C were similar to those when P ointment was applied (Fig. 1 and Fig. 2). In contrast to the creamy composition (cream A) containing only MCT as an oil, transdermal absorption of the main ingredient can be improved when MCT + IPSE (cream B) and MCT + EGS (cream C) that are creamy compositions containing combination of MCT and IPSE or EGS were used.

(5) Efficacy of O/W type creamy compositions (Evaluation of drug efficacy)

Using a mouse contact dermatitis model, efficacy of cream C was examined. Five mice per one group were examined.

On an abdomen of preliminary sheared Balb/c mouse, 0.1 mL of a sensitizer (3 w/v% picryl chloride (PCL)-ethanol solution) was applied to cause sensitization, and after 6 days, each 10 $\mu$L (a total of 20 $\mu$L) of an initiation substance (1 w/v% picryl chloride - acetone solution) was applied on both sides of right auricle of mouse, to initiate allergic reaction.

After 1 hour from initiation of allergic reaction, a placebo, cream C or P ointment was applied on both sides of right auricle (10 mg/ear). Before initiation of allergic reaction (previous value) and after 24 hours from initiation of allergic reaction, thicknesses of the right ear and the left ear were measured by a dial thickness gauge (Mitutoyo Corporation), and an increase of auricle thickness (mm) was calculated.

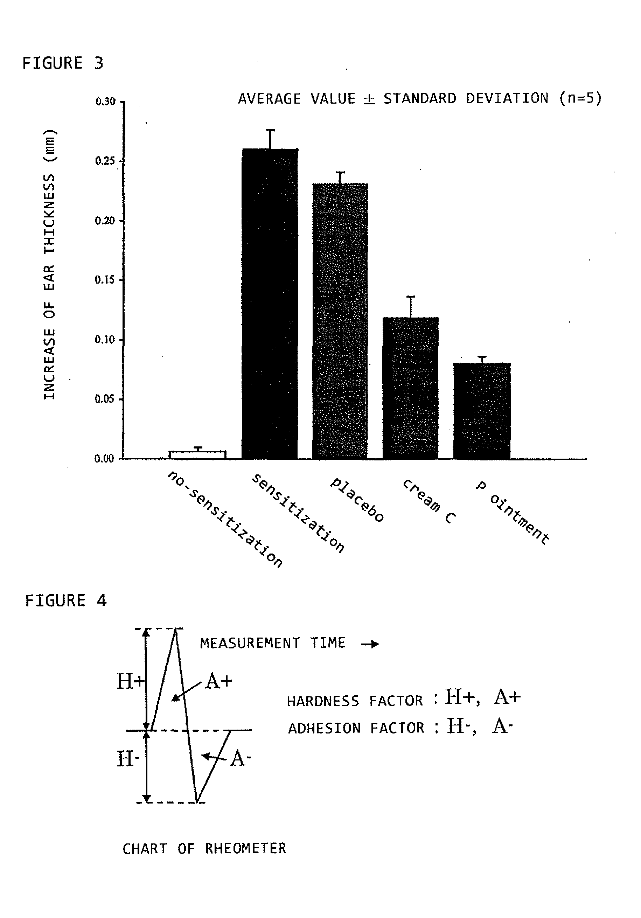

Fig. 3 shows an increase of auricle thickness after 23 hours from application of a placebo (placebo in the drawing), an increase of auricle thickness after 23 hours from application of cream C (cream C in the drawing), and an increase of auricle thickness after 23 hours from application of P ointment (P ointment in the drawing) after conducting sensitization and an initiation treatment. The notation "no sensitization" in the drawing represents an increase of auricle thickness in the group that is subjected to only the initiation treatment but not to sensitization, and "sensitization" represents an increase of auricle thickness in the group that is subjected to only sensitization and initiation treatment. The test method is collectively shown in Table 5. [Table 5]

Table 5 Test design for drug efficacy evaluation of O/W type creamy composition

| Test group | sensitizer application | Application of initiation substance | Dosage | Administration time | Administration route | Sex | Number |
|---|---|---|---|---|---|---|---|
| No-sensitization | - | | - | - | - | ♀ | 5 per each group |
| Sensitization | + | + | | | | | |
| Placebo | | | 10 mg/ear | 1 hour after allergy initiation | transdermal | | |
| Cream C | | | | | | | |
| P ointment | | | | | | | |

An average increase of auricle thickness was 6 μm in "No-sensitization" group, 260 μm in "Sensitization" group, 231 μm in "Placebo" group, and 118 μm in "Cream C" group. These demonstrate that cream C greatly suppresses auricle edema induced by PCL in comparison with the placebo.
It is also demonstrated that an increase of auricle thickness of 80 μm in "P ointment" group has no significant statistical difference in comparison with that of "cream C" group, and cream C shows drug efficacy that is comparable to that by P ointment.

(6) Evaluation of stickiness of O/W type creamy composition (Comparison with prior art)

"Stickiness" was evaluated for each of cream C and a composition described as prescription 20 in Patent document 1 (JP 2000-513739W) in the following manner. Prescription 20 is a creamy composition containing 0.1% by weight of FK 506 substance (tacrolimus) as a main ingredient, each 20.0% by weight of isopropyl myristate and diethyl sebacate as oils, 2. 5% by weight of polyoxyethylene [20] sorbitan monooleate as an emulsifying agent, 1.0% by weight of carboxyvinyl polymer (Carbopol 940) as a hydrophilic polymer, an appropriate amount of sodium hydroxide as a pH modifier, and purified water as a balance.

"Stickiness" is considered as a feel caused by adhesion due to vertical motion of a finger with respect to the skin surface [Reference document 1: Masamichi Morita et al., Relation between functionality evaluation and rheological property of cosmetic emulsion, J. Soc. Cosmet. Chem. Jpn. Vol. 24(2), 91-97 (1990)]. In a rheometer, a sample pedestal on which a sample is placed moves vertically, and after an adaptor enters inside the sample, separation is executed. At this time, a chart as shown in Fig. 4 is obtained, and generally, height (H+) and area (A+) in the + direction are represented as hardness factors, and height (H-) and area (A-) in the - direction are represented as adhesion factors. Also, it is reported that there is correlation between A- and stickiness [Reference document 2: Ichiro Iida et al., Research on typing of cosmetics, J. Soc. Cosmet. Chem. Jpn. Vol. 23(4), 295-300 (1990)], and the higher the stickiness of the sample, the larger the A-, or the larger the value of the adhesion. From this, also in the present test, evaluation by a rheometer was conducted for an index of stickiness.

<Measuring condition of rheometer>

Rheometer: CR-500DX (available from Sun Scientific Co., Ltd.) Rheometer analysis software: RHEO DATA ANALYZER for Win (available from Sun Scientific Co., Ltd.)
Adaptor: No.25 (20 mm) (acrylic cylinder, diameter 20 mm) Measurement mode: 20
Measurement rate (moving speed of sample pedestal): 120 mm/min Sample amount: 8 g
Entry distance of adaptor into sample: 2.0 mm

The result is shown in Table 6. As a result of the test, it is demonstrated that cream C according to the present invention is less adhesive and is unlikely to be sticky in comparison with prescription 20 according to the prior art document.

[Table 6]

Table 6. Result of stickiness evaluation test

| Composition | Adhesion ($\times 10^{-4}$J) | Average ($\times 10^{-4}$J) | S.D. ($\times 10^{-4}$J) |
|---|---|---|---|
| Cream C | 4.702 | 4.638 | 0.523 |
| | 5.126 | | |
| | 4.086 | | |
| Prescription 20 | 6.213 | 6.213 | 0.855 |
| | 5.359 | | |
| | 7.068 | | |

[Conclusion]

The result of the above (3) demonstrated that the stability of tacrolimus in composition is improved in the composition containing IPSE or EGS together with MCT than in the composition containing MCT singly. Further, as to the dissolubility of tacrolimus hydrate, using IPSE or EGS having higher dissolubility than MCT together with MCT would be more advantageous than using MCT singly.

The result of the above (4) demonstrated that transdermal absorption is improved in the composition containing IPSE or EGS together with MCT than in the composition containing MCT singly. Further, since the degree of improvement in transdermal absorption differs depending on which one of IPSE and EGS is selected as an oil for use together with MCT, it is found that the absorption of the main ingredient can be controlled.

That is, according to the present invention, by varying the ratio of combinational use (a : b) between (a) MCT and (b) IPSE/EGS, or varying the kind of (b) or the ratio of combinational use (IPSE : EGS), it is possible to control so that tacrolimus is at an optimum skin concentration in diseased skin which is a site of application.

Further, the result of the above (5) demonstrated that the composition according to the present invention has excellent allergy suppressing effect, and the result of the above (6) demonstrated that the composition according to the present invention has better feeling upon use in comparison with the creamy composition disclosed in the prior art document.

INDUSTRIAL APPLICABILITY

According to the present invention, it is possible to provide a creamy composition realizing good stability of tacrolimus and allowing easy control of skin concentration of a main ingredient. The creamy composition according to the present invention is suited particularly as a therapeutic drug of atopic dermatitis.

**Claims**

1. An oil-in-water type creamy composition, the composition comprising:

   (A) tacrolimus, a pharmaceutically acceptable salt thereof, or a pharmaceutically acceptable solvate thereof;
   (B) an oil prepared by mixing (a) a medium-chain fatty acid triglyceride with (b) ethylene glycol salicylate and/or diisopropyl sebacate;
   (C) an emulsifying agent having a HLB value of 12 or more; and
   (D) a hydrophilic polymer, and
   having a pH value of 4 to 7.

2. The composition according to claim 1, containing 5 to 30% by weight of a medium-chain fatty acid triglyceride (a) and 3 to 15% by weight of ethylene glycol salicylate and/or diisopropyl sebacate (b).

3. The composition according to claim 1 or 2, wherein the emulsifying agent (C) having a HLB value of 12 or more is one or more selected from the group consisting of polyoxyethylene hydrogenated castor oil, polyoxyethylene polyoxypropylene alkylether, and polyglycerin fatty acid ester.

4. The composition according to any one of claims 1 to 3, wherein the hydrophilic polymer (D) is one or more selected from the group consisting of carboxyvinyl polymer, hydroxyethyl cellulose, hydroxypropyl cellulose, hydroxypropyl methyl cellulose and hydrophobized hydroxypropyl methyl cellulose.

5. The composition according to any one of claims 1 to 4, containing one or more pH modifiers selected from the group consisting of lactic acid, citric acid, phosphoric acid, sodium hydroxide, potassium hydroxide, sodium lactate, sodium citrate, L-arginine and diisopropanolamine.

FIGURE 1

AVERAGE VALUE ± STANDARD DEVIATION (n=3)

CONCENTRATION IN HUMAN SKIN AFTER 24 HOURS

FIGURE 2

AVERAGE VALUE ± STANDARD DEVIATION (n=3)

CONCENTRATION IN HUMAN CORNEUM AFTER 24 HOURS

FIGURE 3

AVERAGE VALUE ± STANDARD DEVIATION (n=5)

FIGURE 4

MEASUREMENT TIME →

HARDNESS FACTOR : H+, A+
ADHESION FACTOR : H-, A-

CHART OF RHEOMETER

## INTERNATIONAL SEARCH REPORT

| International application No. |
|---|
| PCT/JP2011/066698 |

A. CLASSIFICATION OF SUBJECT MATTER
*A61K31/436*(2006.01)i, *A61K9/107*(2006.01)i, *A61K47/14*(2006.01)i

According to International Patent Classification (IPC) or to both national classification and IPC

B. FIELDS SEARCHED

Minimum documentation searched (classification system followed by classification symbols)
A61K31/436, A61K9/107, A61K47/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | JP 2007-145860 A  (Novartis AG.),<br>14 June 2007 (14.06.2007),<br>entire text<br>& US 2001/0031769 A1    & GB 9421612 A0<br>& EP 786986 A        & WO 1996/013249 A1<br>& DE 19581804 T       & NO 971951 A<br>& AU 3845195 A        & FI 971018 A<br>& BR 9509530 A        & HU 77140 A<br>& AU 714254 B         & CA 2200966 A<br>& CZ 9701232 A        & NZ 295170 A<br>& PL 319599 A         & SK 52097 A<br>& CN 1162259 | 1-5 |

☒ Further documents are listed in the continuation of Box C.      ☐ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 09 August, 2011 (09.08.11) | 16 August, 2011 (16.08.11) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2011/066698

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-513739 A (Fujisawa Pharmaceutical Co., Ltd.), 17 October 2000 (17.10.2000), entire text & US 2002/0032212 A1 & EP 977565 A & WO 1998/036747 A1 & DE 69813542 D & NO 994003 A & AU 6228998 A & BR 9807234 A & HU 979 A & IL 131298 A & CA 2282345 A & HK 1027957 A & ZA 9801391 A & TW 450810 B & AT 237325 T & DK 977565 T & EA 3580 B & ES 2193515 T | 1-5 |
| A | JP 3732525 B1 (Astellas Pharma Inc.), 21 October 2005 (21.10.2005), entire text (Family: none) | 1-5 |
| A | JP 2010-132607 A (Taisho Pharmaceutical Industries, Ltd.), 17 June 2010 (17.06.2010), entire text (Family: none) | 1-5 |
| A | JP 2006-241132 A (Maruho Co., Ltd.), 14 September 2006 (14.09.2006), entire text (Family: none) | 1-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000513739 W

**Non-patent literature cited in the description**

- General Tests, Liquid Chromatography
- **MASAMICHI MORITA et al.** Relation between functionality evaluation and rheological property of cosmetic emulsion. *J. Soc. Cosmet. Chem. Jpn.,* 1990, vol. 24 (2), 91-97
- **ICHIRO IIDA et al.** Research on typing of cosmetics. *J. Soc. Cosmet. Chem. Jpn.,* 1990, vol. 23 (4), 295-300